# EUROPÄISCHE PATENTSCHRIFT

(11) **EP 1 714 620 B1**
(45) Veröffentlichungstag und Bekanntmachung des Hinweises auf die Patenterteilung: **09.11.2016**
(21) Anmeldenummer: 06008004.1
(22) Anmeldetag: 18.04.2006
(51) Int. Cl.: A61B 18/22, G02B 6/36

(54) **Lichtleitfaser mit einem Steckerteil**
Lightguide having a connector
Guide de lumière avec un connecteur

(30) Priorität: 18.04.2005 DE 102005017798
(43) Veröffentlichungstag der Anmeldung: 25.10.2006
(73) Patentinhaber: Dornier MedTech Laser GmbH, 82234 Wessling (DE)
(72) Erfinder: Hiereth, Werner, 82205 Gilching (DE); Durian, Oliver, 86399 Bobingen (DE)
(74) Vertreter: Grünecker Patent- und Rechtsanwälte PartG mbB

(56) Entgegenhaltungen:
- DE-U1- 9 013 085
- US-A- 4 519 390
- US-A- 4 781 185
- US-B1- 6 567 582
- US-B1- 6 829 427

## Beschreibung

Die Erfindung betrifft eine Lichtleitfaser mit einem Steckerteil.

Derartige Lichtleitfasern werden in der Medizintechnik zum Leiten von (Laser-)Licht beispielsweise zur Behandlung von Krebsgeschwüren oder Ähnlichem eingesetzt.

Aus der US 5,364,391 ist eine Lichtleitfaser bekannt, die ein Fingerteil aufweist, wobei die Faser in einen Koppler eingesteckt und das andere Ende der Faser zur Behandlung verwendet werden kann. Oft weisen die Fasern an ihrem dem Stecker entgegengesetzten Ende spezielle Formen, oder Besonderheiten auf, die beispielsweise eine seitliche Lichtabstrahlung oder ähnliches bewirken. Diese Fasern sind dadurch recht teuer.

Da die Fasern nach der Behandlung kontaminiert sind, können sie nicht wiederverwendet werden, sondern müssen entsorgt werden. Versuche, stark kontaminierte Fasern zu sterilisieren, haben gezeigt, dass eine ausreichende Keimabtötungsrate innerhalb gängiger Zeiten (3-5 Minuten) nicht möglich ist.

Aus US 6,567,582 ist eine Lichtleitfaser gemäß dem Oberbegriff des Anspruchs 1 bekannt. Aufgabe der vorliegenden Erfindung ist es, eine Lichtleitfaser mit einem Steckerteil und Griffteil zu schaffen, die besser gereinigt und/oder desinfiziert werden kann.

Diese Aufgabe wird durch eine Lichtleitfaser nach Anspruch 1 gelöst.

Vorteilhafte Ausführungsformen sind in den abhängigen Ansprüchen offenbart.

Die Lichtleitfaser mit einem Steckerteil ist so ausgebildet, dass sie gut gereinigt und/oder desinfiziert und/oder sterilisiert werden kann. Zur maschinellen Reinigung und/oder Desinfektion und/oder Sterilisation werden beispielsweise Vorrichtungen eingesetzt, die gerichtete Flüssigkeitsströmungen erzeugen, um so Verschmutzungen und Bakterien zu entfernen. Dazu können die Lichtleitfasern beispielsweise in Reinigungsbecken oder -kammem eingebracht werden, in denen eine Reinigungs-flüssigkeit umgewälzt, versprüht oder in Strahlform auf die Lichtleitfaser gerichtet wird. Auch ist es möglich, die Lichtleitfasern mit dem Steckerteil oder einem Stecker an einer bestimmten Stelle zu fixieren und einen oder mehrere gezielte Reinigungsflüssigkeitsstrahlen auf die Lichtleitfaser, das Steckerteil oder den Stecker zu richten.

Zur Reinigung und/oder Desinfektion und/oder Sterilisation sind Autoklaven möglich.

Die Lichtleitfaser ist in einem Steckerteil aufgenommen. Dazu weist das Steckerteil eine Ausnehmung wie beispielsweise eine zentrale Bohrung auf. Auch kann an dem Steckerteil an der Stelle, an der die Lichtleitfaser aus dem Steckerteil heraustritt, eine Vertiefung vorgesehen sein. Dies kann z. B. bei der Einkopplung von Licht aus Hochleistungslichtquellen erforderlich sein, um eine Zerstörung der Faser im Einkopplungsbereich zu verhindern.

An dem Ende der Ausnehmung also z. B. am Ende der Bohrung können sich zwischen Lichtleitfaser und dem Steckerteil leicht Keime festsetzen, die jedoch durch den Fluiddurchgang, der am Ende der Ausnehmung beginnt und durch den ein Fluid hindurchtreten kann, leicht entfernt werden können.

Durch eine Öffnung einer eventuellen Sackgasse nach hinten kann ein Durchgang geschaffen werden, wodurch die Sackgasse entfällt. Eine Reinigung und/oder Desinfektion und/oder Sterilisation ist somit gut möglich.

Der Fluiddurchgang verläuft vorzugsweise zu einem guten Stück entlang der Lichtleitfaser, dadurch kann der Bereich entlang der Lichtleitfaser gut gereinigt und/oder desinfiziert und/oder sterilisiert werden.

Weiter ist es vorteilhaft, wenn der Fluiddurchgang seitlich der Lichtleitfaser austritt, da dann das eintretende Reinigungsfluid in eine andere Richtung ausgegeben werden kann, als die Richtung, aus der beispielsweise ein Fluidstrahl mit entsprechendem Druck auftrifft. Die seitlich Ableitung hat weiterhin den Vorteil, dass der Durchgang nicht durch das gesamte Steckerteil hindurchgehen muss, was jedoch auch möglich ist.

Um schwer zugängliche Hohlräume zu vermeiden, ist das Steckerteil vorzugsweise mit der Faser verklebt und/oder verkrimpt. Die Klebestellen sind vorzugsweise mit einem elastischen Dichtmittel wie etwa Silikon oder Ähnlichem abgedeckt. Mit dem Verkrimpen lässt sich ein guter Halt zwischen Faser und Steckerteil erreichen. Gegebenenfalls lässt sich die Krimpstelle mit einem Kleber zusätzlich abdichten.

Für eine bessere Handhabung weist die Lichtleitfaser ein geeignetes Griffteil auf.

Das Griffteil kann beispielsweise mit dem Steckerteil verschnappt werden. Dies erlaubt eine recht einfache Konstruktion.

Die Lichtleitfaser weist einen Stecker mit einem Griffteil auf, das zwei Endöffnungen hat, durch die der Lichtweg führt und die durchgängig miteinander verbunden sind. Dadurch kann die Bildung von Sackgassen vermieden werden, was die Reinigung erleichtert und/oder zu einer besseren Desinfizierbarkeit und/oder Sterilisierbarkeit führt.

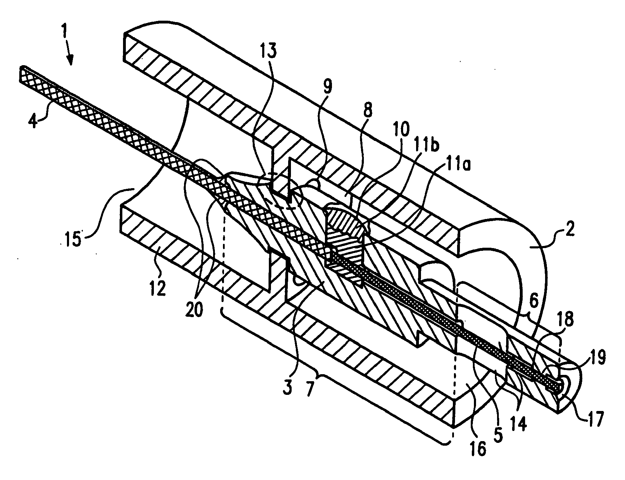

In der beiliegenden Figur ist eine Lichtleitfaser in einer dreidimensionalen schematischen Schnittansicht dargestellt.

Die Lichtleitfaser 1 ist mit einem in der Figur dargestellten Stecker versehen. Die Lichtleitfaser 1 weist zwei Bereiche 4, 5 auf, wobei die Faser 1 im Bereich 4 mit einem Schutzmantel umgeben ist, der im Bereich 5 entfernt wurde. Dieser Schutzmantel kann beispielsweise aus Kunststoff bestehen. Die Lichtleitfaser 1 ist zwischen den Bereichen 4 und 5 durchgängig ausgebildet.

Die Lichtleitfaser 1 mit den Bereichen 4, 5 ist in einem Steckerteil 3 aufgenommen. Das Steckerteil 3 verfügt über eine zentrale Ausnehmung in Form einer Bohrung, in der die Lichtleitfaser 1 angeordnet ist und die an einem Ende einen größeren Durchmesser hat, als beispielsweise in der Mitte oder am anderen Ende. Dadurch kann der Bereich 4 der Lichtleitfaser 1 mit dem Schutzmantel in das Steckerteil 3 eingeführt werden und dabei an einen Anschlag gelangen, wenn der Schutzmantel an den verjüngten Teil der zentralen Bohrung stößt.

Der Bereich 5 der Lichtleitfaser 1 endet bündig mit dem Steckerteil 3 (in der Figur rechts). Die Lichtleitfaser 1 kann aber auch etwas weiter vorstehen oder etwas zurückgesetzt enden.

Das Steckerteil 3 weist an dem Ende des Bereichs 5 bevorzugterweise eine Vertiefung 17 auf, sodass das Ende des Bereichs 5 etwas frei steht. Dies ist für eine Ankopplung an eine Hochleistungslichtquelle von Vorteil.

Im Abschnitt 7 (Klebeabschnitt) des Steckerteils 3 ist die Lichtleitfaser 1 mit dem Steckerteil 3 verklebt. Im Abschnitt 6 (Führungsabschnitt), durch den die Lichtleitfaser 1 mit dem Bereich 5 hindurchtritt, ist der Bereich 5 nicht verklebt. Dadurch, dass hier keine Verklebung vorgesehen ist, dient der Führungsabschnitt 6 zur Aufnahme der Lichtleitfaser 1, ohne dass eine feste Verbindung gegeben ist. Die Lichtleitfaser 1 wird dann durch diesen Abschnitt nur lose geführt. Bei eingesetzter Lichtleitfaser 5 umgibt diese ein Durchgang 18.

Im Abschnitt 7 kann die Lichtleitfaser durch Krimpen oder eine andere mechanische Verbindung mit dem Steckerteil verbunden sein.

Der Abschnitt 6 verfügt über seitlich neben dem Bereich 5 der Lichtleitfaser 1 angeordnete Öffnungen 14. Diese können auch durch eine einzelne Bohrung oder Fräsung durch das Steckerteil 3 hindurch gegeben sein. Dadurch, dass die Lichtleitfaser 1 im Bereich 5 in dem Abschnitt 6 des Steckerteils 3 nicht mit dem Steckerteil 3 verklebt ist, kann so beispielsweise Reinigungsflüssigkeit oder Dampf bei der Vertiefung 17 eintreten, entlang dem Bereich 5 durch die Durchgangsöffnung 18 des Steckerteils 3 bis zur Öffnung 14 gelangen und dort wieder austreten. Dadurch ist entlang dieses Weges eine gute Reinigungs- und/oder Desinfektions- und/oder Sterilisationsmöglichkeit gegeben. Die Reinigungsflüssigkeit oder der Dampf kann auch in umgekehrter Richtung fließen.

Zur Verbesserung der Fluidleitung entlang der Lichtleitfaser 1 in Abschnitt 6 können auf der Innenseite der Durchgangsöffnung 18 des Steckerteils 3 auch Rippen 19, Noppen, oder ähnliche Abstandshalter vorgesehen sein, die zwar die Lichtleitfaser 1 beabstandet zu der Innenseite des Steckerteils 3 halten, jedoch einen leichten Fluiddurchlass durch den Durchgang 18 entlang der Lichtleitfaser 1 ermöglichen. Zwingend nötig sind solche Noppen 19 aber nicht.

An die Öffnung 14 schließt sich der Klebeabschnitt 7 des Steckerteils 3 an. Durch die Verklebung bis zur Öffnung 14 wird die Bildung einer nur schwer zu reinigenden und/oder desinfizierenden und/oder sterilisierenden Sackgasse entlang der Lichtleitfaser 1 vermieden.

Die Öffnungen 14 erstrecken sich in Querrichtung zur Lichtleitfaser 1. Vorzugsweise sind die beiden Öffnungen 14 durchgängig miteinander verbunden, so dass ein Reinigungsfluid oder ein Gasstrom seitlich durch eine Öffnung 14 in das Steckerteil 3 eintreten kann, quer zur Lichtleitfaser 1 durch das Steckerteil 3 hindurchtreten kann und durch die gegenüberliegende Öffnung 14 wieder heraustreten kann. Dadurch ist eine gute Reinigung und/oder Desinfizierbarkeit und/oder Sterilisierbarkeit möglich.

An dem Ende des Steckerteils 3, das in der Figur links dargestellt ist, kann ein elastisches Dichtmittel wie etwa Silikon 20 oder ein elastischer Kleber vorgesehen sein, das die aus dem Steckerteil 3 austretende Lichtleitfaser 1 umschließt, so dass der Bereich zwischen Lichtleitfaser 1 und Steckerteil 3 abgedichtet ist. Auch an der Stelle, an der die Lichtleitfaser 1 in die Öffnung 14 aus dem Steckerteil 3 austritt, kann ein elastisches Dichtmittel (Silikon oder Ähnliches) zum Abdichten vorgesehen sein.

Das Steckerteil 3 kann zusätzlich eine seitliche Öffnung 10 aufweisen, durch die Kleber 11 a bis zum Bereich 4 oder 5 geleitet werden kann, um so die Lichtleitfaser 1 mit dem Steckerteil 3 zu verkleben und diese seitliche Öffnung 10 zu verschließen. Durch entsprechenden Druck kann der Kleber 11 a ausgehend von der Öffnung 10 entlang der Lichtleitfaser 1 bis zur Öffnung 14 und zum in der Figur links dargestellten Ende gedrückt werden, um so ein möglichst vollflächiges Verkleben der Lichtleitfaser 1 mit dem Klebeabschnitt 7 zu erreichen.

Die Kleberöffnung 10 kann beispielsweise an derjenigen Stelle vorgesehen sein, an der der Bereich 4 in den Bereich 5 übergeht. Die Öffnung 10 mit Kleber darin ist vorteilhafterweise mit einem elastischen Dichtmittel 11 b wie z. B. Silikon oder Ähnlichem abgedeckt.

Das Abdecken des Klebers mit dem elastischen Dichtmittel wie Silikon oder Ähnlichem dient dazu, eventuelle Risse in oder bei dem Kleber abzudichten. Risse, die sich gegebenenfalls in dem Kleber oder zwischen dem Kleber und dem Steckerteil 3 bilden sollten, wie dies beispielsweise durch starke thermische Belastung vorkommen kann, wären für eine gute Reinigung und/oder Desinfizierbarkeit und/oder Sterilisierbarkeit nachteilig, da sich dort Keime festsetzen können, die nur schwer entfernt werden können. Daher ist es vorteilhaft, die in Frage kommenden Stellen mit einem elastischen Dichtmittel 11 b, 20 abzudichten. Dieses ist für thermische Belastungen nicht so anfällig, da es elastisch ist und sich den Ausdehnungen der Materialien bei Temperaturänderungen ausreichend anpassen kann.

Auch die mechanische Halteverbindung der Faser 1 an dem Steckerteil 3, z. B. durch Krimpen, kann durch den Kleber und/oder das Dichtmittel abgedichtet werden.

Das Steckerteil 3 ist in einem Griffteil 2 aufgenommen. Das Griffteil 2 hat im wesentlichen die Form eines Rohres 12 und weist zwei Endöffnungen 15, 16 auf. Die Lichtleitfaser 1 tritt mit dem Bereich 4 durch die Endöffnung 15 und mit dem Bereich 5 durch die Endöffnung 16. Das Steckerteil 3 ist mit dem Griffteil 2 über einen Steg 8 verbunden. Der Steg 8 ist kreisscheibenartig ausgebildet und weist eine zentrale Öffnung zur Aufnahme des Steckerteils 3 auf.

Im Bereich 13 kann das Steckerteil 3 mit dem Steg 8 verschnappt werden. Dazu weist das Steckerteil 3 eine rillenartige Vertiefung auf, in die der Steg 8 des Griffteils 2 zum Verschnappen eingeführt werden kann. Dabei wird das Griffteil 2 von in der Figur links mit einer Bewegung nach rechts entlang der Lichtleitfaser 1 über das Steckerteil 3 geschoben. Um dies zu erleichtern, ist an dem schlankeren Ende des Steckerteils 3 eine konusartige Form vorgesehen.

Die Schnappverbindung zwischen Steckerteil 3 und Griffteil 2 ist vorteilhafterweise so ausgestaltet, dass das Griffteil 2 dauerhaft mit dem Steckerteil 3 verbunden ist, d. h. nicht einfach wieder entfernt werden kann. Dadurch wird ein versehentliches Lösen des Griffteils 2 verhindert. Jedoch kann auch ein lösbares Griffteil 2 vorgesehen sein, dass beispielsweise für eine Reinigung und/oder Desinfektion und/oder Sterilisation abgenommen wird, um so eine Reinigung und/oder Desinfektion und/oder Sterilisation noch zusätzlich zu vereinfachen, da Hohlräume weitgehend vermieden werden.

Die zentrale Öffnung des Stegs 8 weist Einbuchtungen 9 auf, so dass diese zentrale Öffnung auch bei eingesetztem Steckerteil 3 offen bleibt. Dadurch sind die Endöffnungen 15, 16 des Griffteils durchgängig miteinander verbunden und beim Reinigen und/oder Desinfizieren und/oder Sterilisieren ist ein Reinigungsmittel- oder Gasstrom durch das Griffteil 2 hindurch möglich, um so eine gute Reinigung und/oder Desinfektion und/oder Sterilisation zu erreichen.

Die in der Figur dargestellte Lichtleitfaser 1 kann mit dem in der Figur dargestellten Stecker in eine Lichtquelle eingesteckt werden, sodass Licht in den Bereich 5 eingekoppelt wird und somit in Verlängerung des Bereichs 4 zur Behandlung zur Verfügung steht.

Das Griffteil 2 verfügt vorzugsweise über einen entsprechenden Verriegelungsabschnitt, mit dem das Griffteil 2 mit einer Lichtquelle verbunden werden kann oder auch mit dem es in einem Reinigungs- und/oder Desinfektions- und/oder Sterilisationsgerät wie beispielsweise einem Autoklaven positioniert werden kann. Dieser Verriegelungsabschnitt kann beispielsweise ein Luer-Lock mit einem zweigängigen Gewinde umfassen.

Das Griffteil 2 kann an seiner Außenseite eine Riffelung, abgeflachte Bereiche oder ähnliches aufweisen, die die Handhabung des Griffteils 2 beim Verschieben oder beim Drehen des Griffteils 2 erleichtern.

## Patentansprüche

1. Lichtleitfaser mit einem Steckerteil (3) mit einem Griffteil (2), wobei das Steckerteil (3) in dem Griffteil (2) aufgenommen ist und das Griffteil (2) zwei Endöffnungen (15, 16) hat, durch die der Lichtweg führt,
wobei das Steckerteil (3) zur Aufnahme der Lichtleitfaser (1) eine Ausnehmung, wie etwa ein Bohrung, aufweist, und wobei ein Fluiddurchgang zwischen der Lichtleitfaser (1) und dem Steckerteil (3) vorgesehen ist, der bei dem Ende der Ausnehmung beginnt und durch den ein Fluid hindurchtreten kann,
**dadurch gekennzeichnet,**
**dass** das Griffteil (2) rohrförmig ist, wobei die zwei Endöffnungen (15, 16) des Griffteils (2) auch bei aufgenommenen Steckerteil (3) durchgängig miteinander verbunden sind, um ein Passieren eines Reinigungsmittel- oder Gasstroms durch das Griffteil (2) hindurch zu ermöglichen.

2. Lichtleitfaser nach Anspruch 1, **dadurch gekennzeichnet, dass** die Lichtleitfaser (4) in eine Endöffnung (15) in das Griffteil (2) eintritt und das Licht der Lichtleitfaser (5) oder die Lichtleitfaser (5) durch die andere Endöffnung (16) aus dem Griffteil (2) heraustreten kann.

3. Lichtleitfaser nach Anspruch 1 oder 2, **dadurch gekennzeichnet, dass** sich der Fluiddurchgang (18, 14) zumindest teilweise entlang der Lichtleitfaser (5) erstreckt.

4. Lichtleitfaser nach einem der Ansprüche 1 bis 3, **dadurch gekennzeichnet, dass** der Fluiddurchgang (18, 14) seitlich der Lichtleitfaser (5) austritt.

5. Lichtleitfaser nach einem der Ansprüche 1 bis 4, **dadurch gekennzeichnet, dass** das Steckerteil (3) mit der Lichtleitfaser **(1)** verklebt und/oder verkrimpt ist.

6. Lichtleitfaser nach Anspruch 5, **dadurch gekennzeichnet, dass** eine oder mehrere der Außenseiten des Klebers (11a) mit einem elastischen Dichtmittel (11b, 20), vorzugsweise etwa Silikon oder elastischer Kleber, abgedeckt sind.

7. Lichtleitfaser nach einem der Ansprüche 1 bis 6, **dadurch gekennzeichnet, dass** das Griffteil (2) auf das Steckerteil (3) aufgesteckt ist.

8. Lichtleitfaser nach Anspruch 7, **dadurch gekennzeichnet, dass** das Griffteil (2) ein Schnappverbindungselement (8) aufweist, mit dem es mit dem Steckerteil (3) verschnappt werden kann.

## Claims

1. Optical fibre with a connector piece (3) with a gripping piece (2), wherein the connector piece (3) is accommodated in the gripping piece (2) and the gripping piece (2) has two end openings (15, 16) through which the optical path leads,
wherein the connector piece (3) has a recess, such as a bore hole, for accommodating the optical fibre (1) and wherein a fluid channel is provided between the optical fibre (1) and the connector piece (3), beginning at the end of the recess and through which a fluid can pass,
**characterised in that**
the gripping piece (2) is tubular, wherein the two end openings (15, 16) of the gripping piece (2) are also in interconnection with each other when the connector piece (3) is accommodated, in order to allow a passing of a cleaning fluid or a gas flow through the gripping piece (2).

2. Optical fibre according to claim 1, **characterised in that** the optical fibre (4) enters the gripping piece (2) through one end opening (15) and the light of the optical fibre (5) or the optical fiber (5) can leave the gripping piece (2) through the other end opening (16).

3. Optical fibre according to claim 1 or 2, **characterized in that** the fluid channel (18, 14) is disposed at least partially along a length of the optical fibre (5).

4. Optical fibre according to one of claims 1 to 3, **characterised in that** the fluid channel (18, 14) leaves to the side of the optical fibre (5).

5. Optical fibre according to one of the claims 1 to 4, **characterised in that** the connector piece (3) is glued and/or crimped to the optical fibre (1).

6. Optical fibre according to claim 5, **characterised in that** one or more of the exterior sides of the glue (11 a) are covered with an elastic sealing material (11b, 20), preferably silicone or elastic glue, for instance.

7. Optical fibre according to one of the claims 1 to 6, **characterised in that** the gripping piece (2) that is mounted on the connector piece (3).

8. Optical fibre according to claim 7, **characterised in that** the gripping piece (2) has a snap-on connection element (8) with which it can be snapped together with the connector piece (3).

## Revendications

1. Fibre optique formant guide de lumière et comprenant une pièce de connecteur (3) avec une pièce de préhension (2), fibre optique
dans laquelle la pièce de connecteur (3) est logée dans la pièce de préhension (2) et la pièce de préhension (2) possède deux ouvertures d'extrémité (15, 16) à travers lesquelles mène le parcours de la lumière,
dans laquelle la pièce de connecteur (3) présente, pour accueillir la fibre optique (1), un évidement de logement, comme un alésage,
et dans laquelle il est prévu entre la fibre optique (1) et la pièce de connecteur (3), un passage de fluide, qui débute à l'extrémité de l'évidement de logement et à travers lequel peut passer un fluide,
**caractérisée**
**en ce que** la pièce de préhension (2) est de forme tubulaire, lesdites deux ouvertures d'extrémité (15, 16) de la pièce de préhension (2) étant également reliées mutuellement de manière continue lorsque la pièce de connecteur (3) est logée dans la pièce de préhension, en vue de permettre le passage d'un fluide de nettoyage ou d'un flux de gaz de nettoyage à travers la pièce de préhension (2).

2. Fibre optique selon la revendication 1, **caractérisée en ce que** la fibre optique (4) pénètre dans une ouverture d'extrémité (15) dans la pièce de préhension (2), et la lumière de la fibre optique (5) ou la fibre optique (5) peut sortir de l'autre ouverture d'extrémité (16) de la pièce de préhension (2).

3. Fibre optique selon la revendication 1 ou la revendication 2, **caractérisée en ce que** le passage de fluide (18, 14) s'étend au moins partiellement le long de la fibre optique (5).

4. Fibre optique selon l'une des revendications 1 à 3, **caractérisée en ce que** le passage de fluide (18, 14) sort latéralement de la fibre optique (5).

5. Fibre optique selon l'une des revendications 1 à 4, **caractérisée en ce que** la pièce de connecteur (3) est collée et/ou sertie sur la fibre optique (1).

6. Fibre optique selon la revendication 5, **caractérisée en ce que** l'un ou plusieurs des côtés extérieurs du collage (11a) est ou sont recouverts par un agent d'étanchéité élastique (11b, 20), de préférence par exemple un silicone ou une colle élastique.

7. Fibre optique selon l'une des revendications 1 à 6, **caractérisée en ce que** la pièce de préhension (2) est emmanchée sur la pièce de connecteur (3).

8. Fibre optique selon la revendication 7, **caractérisée en ce que** la pièce de préhension (2) comporte un élément de liaison par enclenchement (8) par lequel elle peut être enclenchée avec la pièce de connecteur (3).
